# EUROPÄISCHE PATENTSCHRIFT

(11) **EP 2 164 546 B1**
(45) Veröffentlichungstag und Bekanntmachung des Hinweises auf die Patenterteilung: **26.09.2018**
(21) Anmeldenummer: 08801471.7
(22) Anmeldetag: 19.06.2008
(51) Int. Cl.: A61M 5/30, A61M 5/20

(54) **EINWEGINJEKTOR MIT MINDESTENS EINEM STÜTZSTAB**
DISPOSABLE INJECTOR WITH AT LEAST ONE SUPPORT ROD
INSTRUMENT D'INJECTION À USAGE UNIQUE AVEC AU MOINS UNE BARRE DE SUPPORT

(30) Priorität: 06.07.2007 DE 102007031630
(43) Veröffentlichungstag der Anmeldung: 24.03.2010
(73) Patentinhaber: LTS LOHMANN Therapie-Systeme AG, 56626 Andernach (DE)
(72) Erfinder: MATUSCH, Rudolf, 35041 Marburg (DE)
(74) Vertreter: Siemund, Volker
(86) Internationale Anmeldenummer: PCT/EP2008/004948
(87) Internationale Veröffentlichungsnummer: WO 2009/006985

(56) Entgegenhaltungen:
- EP-A- 1 336 419
- WO-A-01/93926
- WO-A-2005/044344
- WO-A-2006/088513
- WO-A-2007/073839
- US-A- 4 227 528

## Beschreibung

Die Erfindung betrifft einen Einweginjektor mit einem Gehäuse, in dem oder an dem - jeweils zumindest bereichsweise - mindestens ein mechanischer Federenergiespeicher, mindestens eine - zumindest zeitweise wirkstoffbefüllbare
- Zylinder-Kolben-Einheit, mindestens ein Kolbenbetätigungsstempel und mindestens eine Auslöseeinheit angeordnet ist,
- wobei der Kolbenbetätigungsstempel zwischen dem Federenergiespeicher und dem Kolben der Zylinder-Kolben-Einheit positioniert ist,
- wobei der Federenergiespeicher mindestens ein vorgespanntes Federelement umfasst,
- wobei der federbelastete Kolbenbetätigungsstempel einen Stempelteller aufweist,
- wobei der federbelastete Kolbenbetätigungsstempel am Stempelteller über mindestens einen Stützstab am Gehäuse abgestützt ist,
- wobei der einzelne Stützstab jeweils am Gehäuse angeformt ist und einen elastischen Biegebalken darstellt,
- wobei die zwischen einem einzelnen Stützstab und dem Stempelteller gelegene Kontaktzone ein den jeweiligen Stützstab radial nach außen drängendes Keilgetriebepaar darstellt,
- wobei die Stützstäbe an mindestens einem, am Gehäuse gelagerten und in einer Sperrstellung positionierten, Auslöseelement - radial nach außen drückend - anliegen,
- wobei das Auslöseelement - als Teil einer Auslöseeinheit - durch Verschieben in eine - den Kolbenbetätigungsstempel freigebende, keine abstützende Wirkung aufweisende - Auslösestellung bringbar ist.

Aus der DE 36 44 984 A1 ist u.a. ein derartiger injektor bekannt. Er hat einen federvorgespannten Kolbenbetätigungsstempel, dessen rückwärtige Stempelstange an ihrem freien Ende elastische Zughaken aufweist. Die Zughaken halten den Kolbenbetätigungsstempel formschlüssig an einer Kante des Injektorgehäuses fest. Sie haben hierzu nur eine geringe Auflagefläche am Gehäuse. Zum Auslösen des Injektors werden die Zughaken von der sie haltenden Kante geschoben. In der Folge schießt der federvorgespannte Kolbenbetätigungsstempel nach vorn, um eine Injektion durchzuführen.
Die EP 1 336 419 A1 und WO 2005/044344 A1 beschreiben jeweils einen Nadel-Injektor mit einer Nadelschutzvorrichtung, dessen Kolbenbetätigungsstempel ein Zugstab bzw. eine Zughülse ist. Der Kolbenbetätigungsstempel, der von einer bei der Injektion den Kolben antreibenden Schraubendruckfeder auf Zug belastet wird, stützt sich an gehäuseseitigen Stützelementen ab. Dabei liegen die Stützelemente am kleinsten Durchmesser des Kolbenbetätigungsstempels an, wodurch sich in der Kontaktzone eine besonders große Flächenpressung ergibt. Die EP 1 336 419 A1 offenbart die Merkmale des Oberbegriffs des Anspruchs 1. Der vorliegenden Erfindung liegt daher die Problemstellung zugrunde, einen modular aufgebauten Einweginjektor zu entwickeln, der bei geringer Baugröße nur wenige Bauteile aufweist und bei einfacher Handhabung eine sichere Lagerung und Funktion gewährleistet.
Diese Problemstellung wird mit den Merkmalen des Hauptanspruchs gelöst. Dazu ist der Stempelteller zur Abstützung des Federenergiespeichers vorgesehen, und ist der Durchmesser des Kolbenschiebers etwas kleiner als der Innendurchmesser des Zylinders der Zylinder-Kolben-Einheit und ist der Durchmesser des Stempeltellers einige Zehntel Millimeter kleiner als der Innendurchmesser des Gehäuses, und ist das Auslöseelement eine außen am Gehäuse gleitgelagerte Schiebehülse.
Mit der Erfindung wird hier beispielsweise ein nadelfreier Einmalinjektor vorgestellt, dessen Kolbenbetätigungsstempel bei einem Auslösevorgang des Einweginjektors freigegeben wird. Dazu wird zum Vorspannen und Halten des Federenergiespeichers der Kolbenbetätigungsstempel über mindestens einen am Gehäuse angeordneten oder im Gehäuse integrierten Stützstab formschlüssig gehalten. Der oder die Stützstäbe werden von einem Auslöseelement bis zum Gebrauch des Einweginjektors in ihrer Sperrposition gehalten. Zum Auslösen des Injektors werden der oder die Stützstäbe freigegeben, so dass sich der Kolbenbetätigungsstempel - unter der Wirkung des Federenergiespeichers - zumindest annähernd parallel zur Mittellinie des Einweginjektors bewegen kann.

Weitere Einzelheiten der Erfindung ergeben sich aus den Unteransprüchen und den nachfolgenden Beschreibungen schematisch dargestellter Ausführungsbeispiele.
- Figur 1:: Einweginjektor mit zwei Stützstäben und Hebelsicherung;
- Figur 2:: wie Figur 1, jedoch entsichert und betätigt;
- Figur 3:: wie Figur 2, jedoch nach dem Medikamentenausstoß;
- Figur 4:: Einweginjektor mit zwei Stützstäben und Banderolensicherung;
- Figur 5:: Einweginjektor mit zwei in Sperrstellung verformten Druckstäben und Auslösehebel;
- Figur 6:: Querschnitt A-A zu Figur 5 und 8;
- Figur 7:: Querschnitt B-B zu Figur 5;
- Figur 8:: wie Figur 5, jedoch 90 Winkelgrade geschwenkt;
- Figur 9:: wie Figur 5, jedoch entsichert;
- Figur 10:: wie Figur 8, jedoch entsichert und betätigt;
- Figur 11:: Dimetrische Ansicht zu Figur 5;
- Figur 12:: Einweginjektor mit zwei in Sperrstellung verformten Druckstäben und Banderolensicherung;
- Figur 13:: wie Figur 12, jedoch durch Entfernen der Banderole entsichert;
- Figur 14:: wie Figur 13, jedoch betätigt;
- Figur 15:: Seitenansicht des Injektors;
- Figur 16:: Dimetrische Ansicht zu Figur 15.

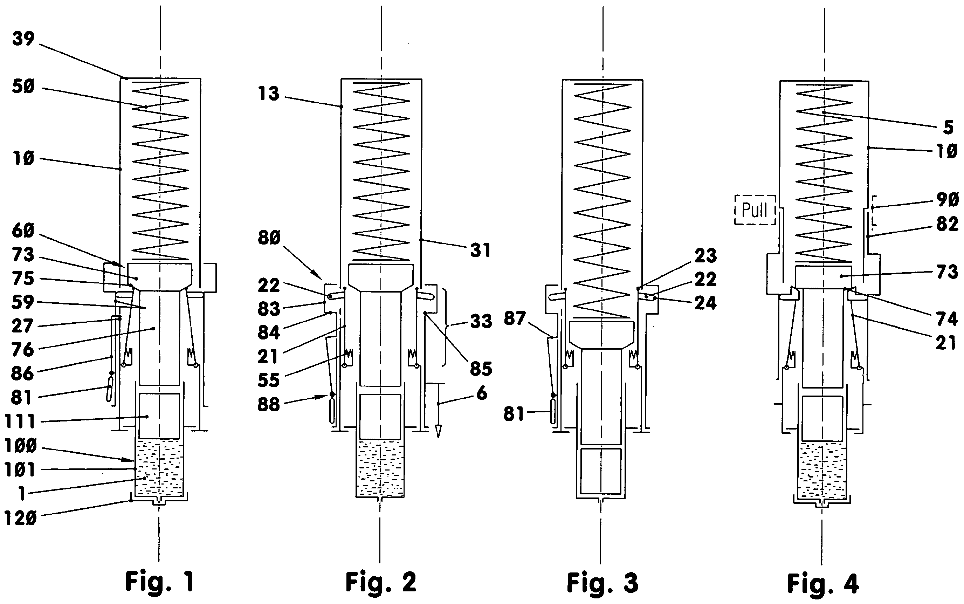

Die Figuren 1 bis 3 zeigen eine vereinfachte Prinzipskizze eines Einweginjektor-Typs mit einem dauergeladenen Federenergiespeicher in drei verschiedenen Auslösezuständen. Der gezeigte Einweginjektor besteht aus einem Gehäuse (10), einer z.B. mit einer Injektionslösung vorbefüllten Zylinder-Kolben-Einheit (100), einem Kolbenbetätigungsstempel (60) und einer Schraubendruckfeder (50) als Federenergiespeicher. Zudem sind am Gehäuse (10) ein Auslöseelement (82) und ein Sicherungselement (86, 90) angeordnet. Die Zylinder-Kolben-Einheit (100) ist vorn mit einer Schutzkappe (120) verschlossen.

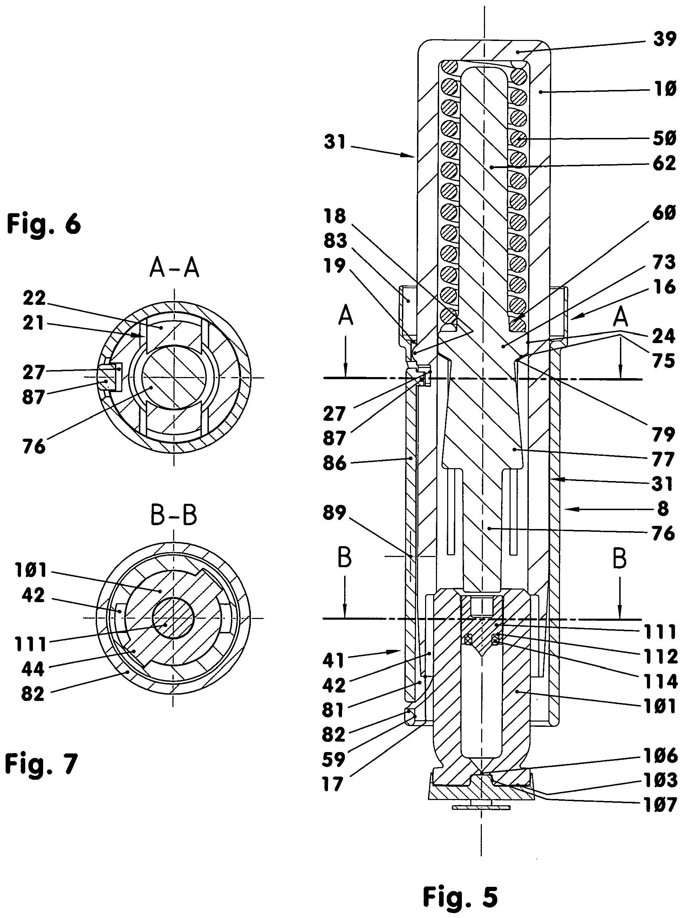

Das Gehäuse (10) ist ein topfförmiger, unten offener Hohlkörper mit obenliegendem Boden (39). Im mittleren Bereich, dem Mantelbereich (31), hat das Gehäuse (10) z.B. zwei einander gegenüberliegende fensterartige Durchbrüche (33). Am jeweils unteren Rand eines Durchbruches (33), vgl. Figur 2, rechte Injektorseite, ist jeweils ein Stützstab (21) gelenkig gelagert. Auf der linken Seite des Gehäuses (10) ist der Durchbruch (33) in den Prinzipskizzen kleiner abgebildet, um den sichernden Auslösehebel (86, 87) darstellen zu können. Der Auslösehebel (86, 87) sitzt in der Regel um z.B. 90 Winkelgrade versetzt im Gehäuse (10), vgl. Figur 6.

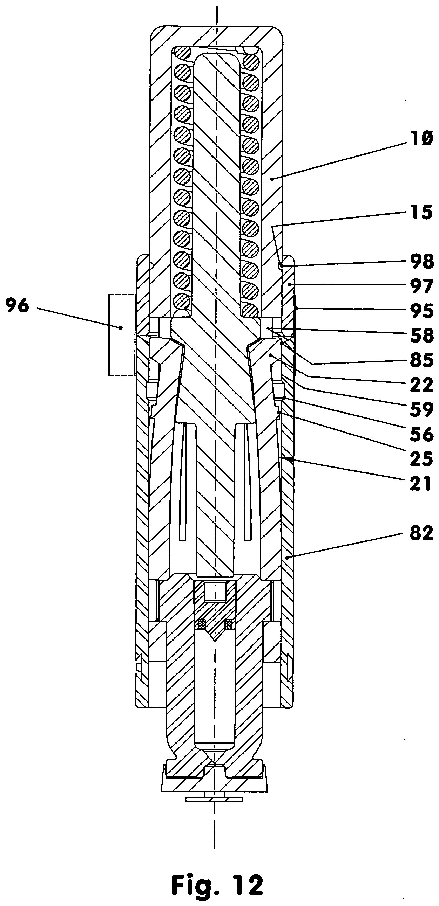

Die Stützstäbe (21) sind hier nur beispielhaft in Schwenkgelenken gelagert und über Federelemente (55) am Gehäuse (10) abgestützt. Die Federelemente (55) drücken die Stützstäbe (21) zumindest annähernd radial nach außen gegen das Auslöseelement (82), vgl. Figur 1. Dort liegen sie über Nocken (22) am Auslöseelement (82) an. Sind die Stützstäbe (21) am Gehäuse (10) angeformt, vgl. Figuren 5 und 12, so federn sie als elastische Biegebalken (28) nach außen.

Die beiden auf Druck belasteten Stützstäbe (21) halten den Kolbenbetätigungsstempel (60) an dessen Stempelteller (73) in seiner vorgespannten Lage, vgl. Figur 1. Dazu stützen sich die Stützstäbe (21) mit ihren Abstützflächen (23) an der unteren kegelstumpfmantelförmigen oder sphärisch gekrümmten Stirnseite (74) des Stempeltellers (73) ab. Die Größe der jeweiligen Kontaktfläche zwischen einer Abstützfläche (23) und der entsprechenden Stirnseitenfläche (74) liegt im Bereich von 2 bis 20 mm².

Auf der der Mittellinie (5) abgewandten Seite weist jeder Stützstab (21) an seinem Nocken (22) eine Anlagefläche (24) auf.

Im unteren Bereich des Gehäuses (10) befinden sich Halteelemente zur Befestigung der Zylinder-Kolben-Einheit (100).

Die Zylinder-Kolben-Einheit (100) besteht im Ausführungsbeispiel aus einem, mit einer Injektionslösung (1) befüllten, Zylinder (101), in dem ein Kolben (111) in der hinteren Position sitzt. Oberhalb des Kolbens (111) ist im Gehäuse (10) der Kolbenbetätigungsstempel (60) z.B. so angeordnet, dass er den Kolben zwar nicht berührt, jedoch mit seinem unteren Ende im oberen Bereich des Zylinders (101) seitlich geführt wird.

Nach Figur 1 ist die untere Hälfte des Gehäuses (10) von dem hülsenartigen Auslöseelement (82) umgeben. Das Auslöseelement (82) ist auf der radialen Außenfläche (13) des Gehäuses (10) längsverschiebbar gelagert. Es hat im oberen Bereich auf der Höhe der Nocken (22) eine umlaufende Aufweitung (83). Anstelle dieser Aufweitung (83) können bei einem nichtrotationssymmetrischen Auslöseelement (82) pro Stützstab (21) auch partielle Aufweitungen oder nicht abgedeckte Öffnungen vorhanden sein.

Die Aufweitung (83) ist im Bezug auf das Gehäuse (10) genau so positioniert und dimensioniert, dass sie die beim Auslösevorgang zurückweichenden, nach außen gedrängten Stützstäbe (21) mit ihren Nocken (22) aufnehmen kann. Die Innenkontur der Aufweitung (83) ist z.B. ein Kanal mit einer Rücksprungflanke (84), die hier eine zur Mittellinie (5) des Injektors normale Ebene darstellt. Der Übergang zwischen der beispielsweise zylindrischen Innenwandung des Auslöseelements (82) und der Rücksprungflanke (84) ist z.B. als scharfkantige Kante (85) ausgebildet. Nach Figur 1 liegen die Nocken (22) mit ihren außen liegenden Anlageflächen (24) an der Innenwandung (59) des Auslöseelements (82) sichernd an.

Am Auslöseelement (82) ist ein sichernder Auslösehebel (86) befestigt oder angeformt. Der Auslösehebel (86) hat an seinem unteren Ende ein druckknopfähnliches Betätigungselement (81), an seinem oberen Ende eine Rastnase (87) und zwischen den Teilen (81) und (87) ein den Auslösehebel (86) lagerndes Schwenkgelenk (88). Die Rastnase (87) ragt nach Figur 1 sperrend in eine Ausnehmung (27) des Gehäuses (10) hinein.

Der im Gehäuse (10) angeordnete Kolbenbetätigungsstempel (60) ist hier in zwei Bereiche aufgeteilt. Der untere Bereich ist der Kolbenschieber (76). Sein Durchmesser ist etwas kleiner als der Innendurchmesser des Zylinders (101) der Zylinder-Kolben-Einheit (100). Die untere Stirnfläche des Kolbenschiebers (76) wirkt direkt auf den Kolben (111).

Der obere Bereich, der Stempelteller (73), ist eine flache, zumindest bereichsweise zylindrische Scheibe, deren Außendurchmesser einige Zehntel Millimeter kleiner ist als der Innendurchmesser des Gehäuses (10) im Mantelbereich (31). Die untere Stirnseite (74) weist eine um den Kolbenschieber (76) herum angeordnete Bundfläche (75) auf. Sie hat die Form eines Kegelstumpfmantels, dessen Spitzenwinkel ca. 100 bis 130, vorzugsweise 120 Winkelgrade beträgt. Die gedachte Spitze des Kegelstumpfmantels liegt auf der Mittellinie (5) im Bereich des Kolbenschiebers (76).

Der Kolbenschieber (76) kann selbstverständlich auch als separates, vom Stempelteller (73) getrenntes, Bauteil ausgeführt sein. Hierzu ist er dann an der Innenwandung des Gehäuses (10) geführt.

Zwischen dem Stempelteller (73) und dem oben liegenden Boden (39) des Gehäuses (10) sitzt vorgespannt die Schraubendruckfeder (50). Die Federkraft wird über den Stempelteller (73) auf die Stützstäbe (21) übertragen. Aufgrund der Neigung der Bundfläche (75) werden die Stützstäbe (21) keilgetriebeartig radial nach außen gedrängt. Die Auslösehülse (82) stützt diese Radialkraft dauerhaft ab.

Zum Betätigen des Einweginjektors wird - nach dem Entfernen der Schutzkappe (120) der Zylinder-Kolben-Einheit (100) - der Einweginjektor auf der Injektionsstelle positioniert. Dabei wird der Einweginjektor zwischen dem Daumen und den restlichen Fingern der den Injektor haltenden Hand fixiert. Der Daumen liegt auf dem Betätigungselement (81) auf. Ist das Betätigungselement (81) oder sind Teile des Auslösehebels z.B. durch ein Klebeetikett oder dergleichen zusätzlich gesichert, so sind diese vor dem folgenden Betätigungsschritt zu entfernen.

Durch das Drücken des Betätigungselements (81) schwenkt die Rastnase (87) des Auslösehebels (86) aus der Gehäuseausnehmung (27) heraus. Die sichernde Verrastung zwischen dem Gehäuse (10) und dem hülsenartigen Auslöseelement (82) ist aufgehoben. Nun kann das Auslöseelement (82) in Richtung der Zylinder-Kolben-Einheit (100) verschoben werden. Bei diesem Vorgang gleitet das Auslöseelement (82) auf der Außenwandung (13) des Gehäuses (10) linear nach unten, also in Richtung der Injektionsstelle. Die Anlageflächen (24) der Stützstäbe (21) rutschen über die Kante (85) und springen unter der Kraft des Federelements (50) entsichernd radial nach außen in die Aufweitung (83). Der Kolbenbetätigungsstempel (60) schnellt ungehindert nach unten, vgl. Figur 3. Der Zylinder (100) wird entleert.

Anstelle einer linearen Gleitbewegung des Auslöseelements (82) auf dem Gehäuse (10) kann auch eine schraubenförmige Bewegung vorgesehen werden. In diesem Fall werden das Auslöseelement (82) und das Gehäuse (10) z.B. über einen Kulissenstein und eine Kulisse aneinander geführt. Ggf. kann das Auslösen auch durch eine reine Schwenkbewegung zwischen dem Gehäuse (10) und dem Auslöseelement (82) realisiert werden. Die Schwenkachse wäre hier die Mittellinie (5).

Die Figur 4 zeigt eine Variante, die keinen Auslösehebel (86) benötigt. Statt dessen ist das Auslöseelement (82) nach oben verlängert. Das obere, verlängerte Ende des Auslöseelements (82) ist mit einer Banderole (90) am Gehäuse (10) gesichert. Die abreiß- oder auftrennbare Banderole (90) verklebt die Teile (10) und (82) temporär. Zum Entsichern des Injektors wird die Banderole (90) abgezogen oder so aufgetrennt, dass die Klebeverbindung zwischen dem Gehäuse (10) und dem Auslöseelement (82) aufgehoben ist.

Bei dieser Variante ist die Bundfläche (75) des Stempeltellers (73) plan ausgeführt. Die Bundfläche (75) ist normal zur Mittellinie (5) orientiert. Sie kontaktiert über eine abgerundete Kante die oberen Stirnflächen der Stützstäbe (21). Diese Stirnflächen sind keilförmig, kegelstumpfmantelförmig oder sphärisch gekrümmt. Die Krümmung ist jeweils so orientiert, dass auf die Stützstäbe (21) - wie bei der Variante nach den Figuren 1 bis 3 - eine radial nach außen wirkende Kraft auftritt.

Die Figuren 5 bis 11 zeigen eine Ausführungsform des in den Figuren 1 bis 3 beschriebenen Prinzips. Hier ist das tragende Bauteil ein einteiliges Gehäuse (10). Es wird z.B. aus einem glasfaserverstärkten Polyamid durch Spritzgießen gefertigt. Das Gehäuse (10) hat eine weitgehend rohrförmige Gestalt und ist in zwei Funktionsbereiche aufgeteilt, das ist zum einen der obere Mantelbereich (31) und zum anderen der untere Fixierbereich (41).

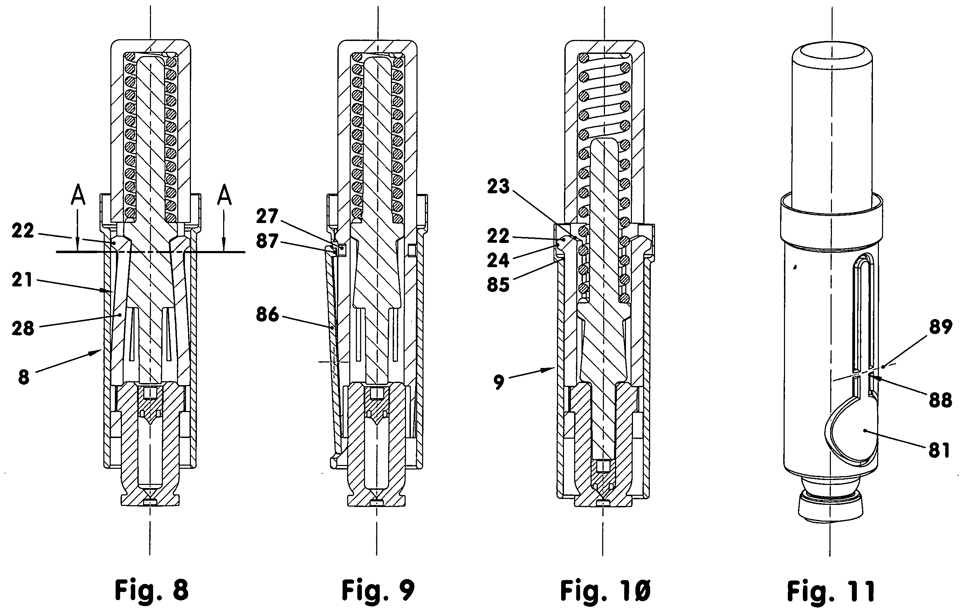

Der im wesentlichen rohrförmige Mantelbereich (31) ist oben durch einen z.B. ebenen Boden (39) verschlossen. In der unteren Hälfte des Mantelbereichs (31) befindet sich, vgl. Figur 8 und 10, zwei einander gegenüberliegende, angeformte Zughaken (21). Die Anformstelle für die Zughaken (21) liegt knapp oberhalb des Fixierbereichs (41). Zur Ausbildung des jeweiligen Stützstabs (21) befindet sich im Mantelabschnitt (31) ein schmaler, zumindest annähernd u-förmiger Spalt, der den einzelnen Stützstab seitlich und oben umgibt. Der Stützstab (21) hat auf ca. 80% seiner Länge die Wandstärke und die Krümmung der Wandung des Gehäuses (10). Dieser Bereich hat u.a. auch die Funktion eines federelastischen Biegebalkens (28). Er hat einen sichelförmigen Querschnitt.

Ggf. kann ein Teil dieses Biegebalkens (28) auch mit einem rechteckigen Querschnitt ausgestattet sein, um bei der Nutzung auftretende Biegespannungen im Biegebalkenrandbereich zu reduzieren. In der Figur 10 ist der Stützstab (21) im unverformten Zustand dargestellt.

Das hier obere freie Ende des einzelnen Stützstabs (21), vgl. Figuren 8 und 10, wird durch den radial nach außen abstehenden Nocken (22) gebildet. Letzterer hat zumindest eine Abstützfläche (23) und eine Anlagefläche (24). Nach Figur 8 liegt auf der Abstützfläche (23) der Stempelteller (73) des gespannten Einweginjektors über seine Bundfläche (75) auf. Die Abstützfläche (23), die hier die Funktion einer Keilfläche erfüllt, hat die Form eines Kegelstumpfmantels mit einem Spitzenwinkel von 120 Winkelgraden.

Ggf. haben die Stützstäbe (21) oder die Bundfläche (75) zumindest im Kontaktbereich eine keramische Panzerung. Im Ausführungsbeispiel nach Figur 5 ist die Bundfläche (75) durch eine z.B. aufgeklebte, mittig geteilte und kegelstumpfmantelförmige Unterlagscheibe (79) verstärkt.

Die Anlagefläche (24) der Nocken (22) der unverformten Stützstäbe (21) ist Teil eines Zylindermantels, dessen Durchmesser z.B. 3 bis 4 Millimeter größer als der Außendurchmesser des Gehäuses (10) ist. Die Anlagefläche (24) kontaktiert bei gespanntem Einweginjektor die Innenwandung (59) des hülsenartigen Auslöseeelements (82). Ggf. hat - zur Minimierung der Flächenpressung - die Anlagefläche (24) eine Krümmung, die der Innenwandung (59) entspricht.

Nach Figur 5 hat das Gehäuse (10) ca. mittig, vgl. Schnittlinie A-A, eine Ausnehmung (27), in die die Rastnase (87) des Auslösehebels (86) hineingreift. Figur 6 zeigt den Eingriff im Querschnitt. In diesem Querschnitt sind auch die beiden Stützstäbe (21) mit den Nocken (22) zu erkennen.

Oberhalb der Ausnehmung (27) befindet sich ein Verdrehsicherungssteg (18). Er greift in eine entsprechende Nut (19) des Auslöseelements (82) als Verdrehsicherung ein.

Unterhalb des Mantelabschnitts (31) befindet sich der Fixierbereich (41) zur Aufnahme der einbaubaren Zylinder-Kolben-Einheit (100). Der Fixierbereich (41) ist Teil eines Bajonettverschlusses. Dazu sind an seiner Innenwandung zwei oder mehrere winkelförmige Kanäle (42) angeordnet, vgl. Figur 7. Die Kanäle (42) führen von der unteren Gehäusestirnseite (17) aus vertikal nach oben und gehen nach wenigen Millimetern Länge jeweils in einen kurzen horizontalen Kanalabschnitt über. Ggf. bilden die querliegenden Kanalanteile eine radial durchgehende Ausnehmung.

Im Fixierbereich (41) ist der Zylinder (101) über z.B. zwei oder mehrere Bajonettzapfen (44) eingesetzt und fixiert, vgl. Figur 7. Ggf. befindet sich im horizontalen Kanalabschnitt oder an zumindest einem Teil der Bajonettzapfen (44) ein oder mehrere Rastelemente, die ein Lösen des Bajonettverschlusses - also ein Entfernen des Zylinders (101) - verhindern.

Der Zylinder (101) ist z.B. ein dickwandiger Topf. In der beispielsweise zylindrischen Bohrung des Zylinders (101) sitzt der stangenlose Kolben (111). Der Kolben (111) hat an seiner vorderen, zumindest annähernd kegelig gestalteten Stirnfläche eine axiale Ringnut (112) zur Aufnahme eines Dichtringes (114) oder einer dauerelastischen Dichtmasse. In der rückseitigen Stirnfläche des Kolbens (111) ist ggf. eine z.B. zylindrische Metallplatte eingelassen.

Im Zentrum der Bohrung des Zylinders (101), dessen Zylinderboden der Kontur der vorderen Kolbenstirnseite zumindest annähernd angepasst ist, befindet sich eine kurze zylindrische, düsenartige Bohrung (106). Ihr Durchmesser beträgt ca. 0,1 bis 0,5 Millimeter. Diese Bohrung (106) ist ein- bis fünfmal so lang wie ihr Durchmesser. Sie endet in einer zylindrischen Ausnehmung (107) der bodenseitigen, äußeren Stirnfläche (103) des Zylinders (101).

Im Fixierbereich (41) ist die Außenwandung des Gehäuses (10) kegelstumpfmantelförmig ausgeführt. Die Wandstärke verjüngt sich zur Stirnseite (17) hin um ca. 20%, damit der Druckknopf (81) beim Betätigen zurückweichen kann.

Zwischen dem Kolben (111) und dem Boden (39) ist der Federenergiespeicher (50) bzw. die Antriebseinheit des Einweginjektors angeordnet. Der Federenergiespeicher (50) ist eine Schraubendruckfeder, die auf dem Kolbenbetätigungsstempel (60) mit dem Stempelteller (73) angeordnet ist. Mittels des Stempeltellers (73) stützt sich der federkraftbelastete Kolbenbetätigungsstempel (60) an den Stützstäben (21) des Gehäuses (10) ab.

Der Kolbenbetätigungsstempel (60) hat oberhalb des Stempeltellers (73) einen Führungszapfen (62). Letzterer führt die Schraubendruckfeder (50). Unterhalb des Stempeltellers (73) befindet sich zentral in der Verlängerung des Führungszapfens (62) ein Kolbenschieber (76), der bei einer Betätigung des Einmalinjektors auf den Kolben (111) wirkt. Der obere Bereich des Kolbenschiebers (76) weist eine konische Verbreiterung (77) auf, deren Länge der halben Länge des Kolbenschiebers (76) entspricht. Der Durchmesser der Verbreiterung (77) nimmt mit zunehmender Entfernung vom Stempelteller (73) zu. An der Verbreiterung (77) liegen bei gespanntem Injektor die Stützstäbe (21) an. Auf diese Weise wird einem Einknicken der dauernd belasteten Stützstäbe (21) entgegengewirkt.

Das teilweise das Gehäuse (10) und die Zylinder-Kolben-Einheit (100) umgebende Auslöseelement (82) ist hier ebenfalls eine Auslösehülse. Die im Wesentlichen zylindrische, z.B. aus ABS gefertigte, Auslösehülse (82) hat an ihrem oberen Ende eine ringförmige radiale Aufweitung (83), die nach dem Auslösen des Einweginjektors die Nocken (22) der Stützstäbe (21) aufnimmt, vgl. Figur 10.

Im darunter liegenden, zumindest annähernd zylindrischen Bereich ist der Auslösehebel (86) integriert, vgl. Figur 11. Letzterer ist über ein Schwenkgelenk (88) mit der Auslösehülse (82) verbunden. Der Auslösehebel (86) bildet zusammen mit dem Betätigungselement (81) eine Wippe, die im Bereich des Schwenkgelenks (88) ihre Schwenkachse (89) hat. Wird der Auslösehebel (86) durch ein Drücken auf das Betätigungselement (81) gegen das Gehäuse (10) gepresst, verlässt am anderen Ende des Auslösehebels (86) die Rastnase (87) entsichernd die Ausnehmung (27), vgl. Figur 9.

Die Figur 9 zeigt den Einweginjektor mit betätigtem Auslösehebel, also entsichert. In Figur 10 ist der Injektor mit nach unten geschobenem Auslöseelement (82) dargestellt.

Mit dem Nachuntenschieben der Auslösehülse (82) rutschen die Nocken (22), vgl. Figur 10, über die Kante (85) nach außen in die Aufweitung (83). Die Stützstäbe (21) biegen sich elastisch nach außen in ihre eigentliche Ausgangslage. Die nun nicht mehr verformten Stützstäbe (21) geben den Kolbenbetätigungsstempel (60) frei, so dass der Kolben (111) unter der Wirkung des Federelements (50) ruckartig in den Zylinder (101) eingeschoben wird.

Die Figuren 12 bis 16 stellen einen Stützstab-Injektor ohne Auslösehebel (86) dar. Anstelle des Auslösehebels wird als Originalitätsverschluss und Sicherungselement eine Banderole (95) verwendet, wie sie zum Teil schon aus Figur 4 bekannt ist. Dazu sitzt oberhalb des auf dem Gehäuse (10) längsgeführten hülsenförmigen Auslöseelements (82) ein Haltering (97). Der auf dem Gehäuse (10) gelagerte rohrförmige Haltering (97) hat eine Rastnocke (98), die in eine Haltenut (15) des Gehäuses (10) eingreift. Über die Rastnocke (98) ist der Haltering (97) auf dem Gehäuse (10) axial fixiert. Ggf. werden am Haltering (97) anstelle der Rastnocke (98) ein Ring von Nocken oder sogar ein umlaufender Raststeg verwendet.

Der Haltering (97) hat z.B. eine plane untere Stirnseite, an der sich - nach Figur 12 - unmittelbar das Auslöseelement (82) mit seiner Stirnfläche (58) anschließt. Der Haltering (97) und das Auslöseelement (82) haben in diesem Bereich den gleichen Außendurchmesser.

Die Stirnfläche (58) ist hier eine kegelstumpfmantelförmige Fläche, deren Sitze auf der Mittellinie (5) unterhalb des Halteringes (97) liegt. Der Kegelwinkel beträgt z.B. 120 Winkelgrade. Die Kontaktlinie zwischen der Stirnfläche (58) und der Innenwandung (59) bildet die Kante (85), über die beim Auslösen des Injektors die Nocken (22) rutschen.

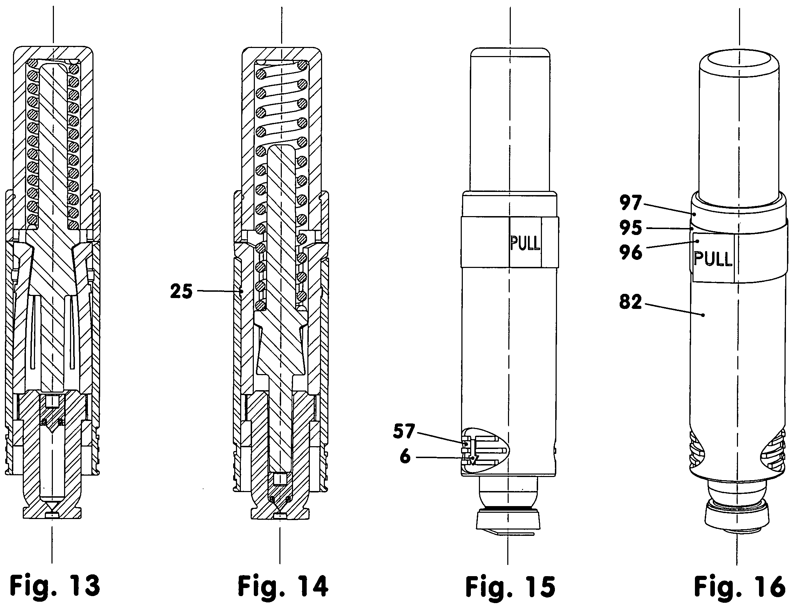

Nach Figur 14 haben die Nocken (22) eine radiale Ausdehnung nach außen, die kleiner ist als die dortige Wandstärke des Auslöseelements (82). Folglich können die Nocken (22) beim Auslösen nicht über die Außenwandung des Auslöseelements (82) überstehen.

Der Haltering (97) und das Auslöseelement (82) sind über eine Banderole (95) miteinander verklebt. Die Banderole (95) ist z.B. ein mit einem Klebstoff einseitig beschichteter Papier- oder Folienstreifen. Der Folienstreifen umgibt z.B. einlagig einmal den Verbund aus Haltering (97) und Auslöseelement (82). Dabei klebt die obere Hälfte des Folienstreifens auf dem Haltering (97) und die untere Hälfte auf dem Auslöseelement (82). Der Folienstreifen ist im Ausführungsbeispiel ca. 2 bis 3 Zentimeter länger als der Umfang des Halteringes (97). Ein beim Verkleben der Teile (82, 97) überstehender Bereich bildet eine Abreißfahne (96). Die zwei bis drei Zentimeter lange Abreißfahne (96) ist beidseitig nicht mit Klebstoff behaftet. Wird die Banderole (95) mit Hilfe der Abreißfahne (96) vom Haltering (97) und dem Auslöseelement (82) - unter einem Lösen der Klebeverbindung - vollständig abgewickelt, so kann das Auslöseelement (82) auf dem Gehäuse (10) vom Haltering (97) nach unten wegbewegt werden.

Das Auslöseelement (82) hat wenige Millimeter unterhalb seiner oberen Stirnseite in seiner Innenwandung (59) eine z.B. umlaufende Arretiernut (56). In die Arretiernut (56) rasten bei einem Auslösen des Injektors spezielle Arretierstege (25) der Stützstäbe (21) ein, vgl. auch Figur 14. Mit dieser Arretierverrastung wird verhindert, dass der Einmal-Injektor nach seinem Gebrauch in seine Einzelteile zerlegt werden kann.

Das Auslöseelement (82) trägt in seinem unteren Bereich auf seiner Außenwandung zwei Riffelungen (57) mit elliptischen Umrandungen. Die Riffelungen bzw. Strukturen sind über eine 180 Winkelgradteilung auf der Außenwandung positioniert. In die Riffelung (57) ist ein nach unten weisender Richtungspfeil (6) integriert.

Bei dieser Ausführungsvariante sind mit Ausnahme des Federelements (50) alle Bauteile rotationssymmetrisch und/oder zu einer auf der Mittellinie (5) gelegenen Ebene spiegelsymmetrisch aufgebaut, was die Montage vereinfacht.

Zum Betätigen des Injektors wird an der Zylinder-Kolben-Einheit (100) die Schutzkappe (120) entfernt und die Banderole (95) zum Entsichern quer zur Injektorlängsrichtung (5) z.B. tangential abgezogen, vgl. Figur 13. Nach dem Aufsetzen auf die Injektionsstelle wird das hülsenförmige Auslöseelement (82) nach unten geschoben. Die Stützstäbe (21) springen nach außen und geben den federbelasteten Stempelteller (73) frei, vgl. Figur 14. Mit der Abgabe des Medikaments über die Zylinder-Kolben-Einheit (100) ist der Injektionsvorgang beendet.

Bei Injektoren, bei denen der Kolbenbetätigungsstempel (60) im Gehäuse (10) - zumindest abschnittsweise - mit geringem Spiel geradgeführt ist und der Kolbenbetätigungsstempel (60) eine ausreichende Biegefestigkeit aufweist, kann anstatt zwei oder mehrerer Stützstäbe (21) auch nur ein einziger Stützstab (21) verwendet werden.

Bei den in den Figuren dargestellten Varianten ist die einzelne Kontaktzone zwischen dem Stützstab (21) und dem Stempelteller (73), als Flächen (23) und (74, 75) ausgeführt, die gleitfähig einander kontaktieren. In einer besonderen Ausgestaltung kann in jeder Fläche (23) der einzelnen Stützstäbe (21) eine Walze gelagert werden, die bei einer Betätigung des Injektors an der Fläche (74, 75) des Stempeltellers wälzgelagert, also reibungsarm, abrollt.

Mit Ausnahme des Federelements (50), einer ggf. vorhandenen Kolbenplatte und der beispielsweise vorhandenen Lagerwalzen der Stützstäbe (21) sind alle Teile der zuvor beschriebenen Einweg-Injektoren aus Kunststoffen oder kunststoff- bzw. gummiähnlichen Werkstoffen gefertigt.

### Bezugszeichenliste:

- 1: Injektionslösung; Medikament
- 5: Mittellinie des Injektors, Längsrichtung
- 6: Auslösebewegungsrichtung von (82), Abwärtsbewegung Richtungspfeil
- 8: Sperrstellung
- 9: Lösestellung, Auslösestellung

- 10: Gehäuse, einteilig
- 13: Außenfläche, zylindrisch
- 15: Haltenut
- 16: Auslösebereich, oben
- 17: untere Gehäusestirnseite
- 18: Verdrehsicherungssteg
- 19: Nut in (82)

- 21: Stützstäbe, Druckstäbe
- 22: Nocken
- 23: Abstützfläche
- 24: Anlagefläche
- 25: Arretierstege
- 27: Ausnehmung für (87)
- 28: Biegebalken

- 31: Mantelbereich
- 33: Durchbrüche
- 39: Boden

- 41: Fixierbereich für die Zylinder-Kolben-Einheit
- 42: Kanäle, winkelförmig
- 44: Bajonettzapfen
- 50: Federelement, Schraubendruckfeder, Federenergiespeicher
- 55: Federelemente an (21)
- 56: Arretiernut von (82)
- 57: Riffelung von (82)
- 58: Stirnfläche von (82)
- 59: Innenwandung von (82)

- 60: Kolbenbetätigungsstempel
- 62: Führungszapfen

- 73: Stempelteller
- 74: Stirnseite, unten; Stirnseitenfläche
- 75: Bundfläche, Stirnseitenfläche
- 76: Kolbenschieber
- 77: Konusverbreiterung
- 79: Unterlagscheibe

- 80: Auslöseeinheit
- 81: Betätigungselement, Druckknopf
- 82: Auslöseelement
- 83: Aufweitung
- 84: Rücksprungflanke
- 85: Kante, scharfkantig
- 86: Auslösehebel, Sicherungselement
- 87: Rastnase
- 88: Schwenkgelenk
- 89: Schwenkachse

- 90: Originalitätsverschluss, Banderole, Sicherungselement
- 95: Originalitätsverschluss, Banderole, Sicherungselement
- 96: Abreißfahne
- 97: Haltering
- 98: Rastnocke
- 100: Zylinder-Kolben-Einheit
- 101: Zylinder
- 103: Stirnfläche
- 106: Bohrung, Düse
- 107: Ausnehmung in der Stirnfläche
- 111: Kolben
- 112: Ringnut
- 114: Dichtring, Dichtung

- 120: Schutzkappe, Klebeversiegelung

## Patentansprüche

1. Einweginjektor mit einem Gehäuse (10), in dem oder an dem - jeweils zumindest bereichsweise - mindestens ein mechanischer Federenergiespeicher (50), mindestens eine - zumindest zeitweise wirkstoffbefüllbare - Zylinder-Kolben-Einheit (100), mindestens ein Kolbenbetätigungsstempel (60) und mindestens eine Auslöseeinheit (80) angeordnet ist,
- wobei der Kolbenbetätigungsstempel (60) zwischen dem Federenergiespeicher (50) und dem Kolben (111) der Zylinder-Kolben-Einheit (100) positioniert ist,
- wobei der Federenergiespeicher (50) mindestens ein vorgespanntes Federelement (50) umfasst,
- wobei der federbelastete Kolbenbetätigungsstempel (60) einen Stempelteller (73) aufweist,
- wobei der federbelastete Kolbenbetätigungsstempel (60) am Stempelteller (73) über mindestens einen Stützstab (21) am Gehäuse (10) abgestützt ist,
- wobei der einzelne Stützstab (21) jeweils am Gehäuse (10) angeformt ist und einen elastischen Biegebalken (28) darstellt,
- wobei die zwischen einem einzelnen Stützstab (21) und dem Stempelteller (73) gelegene Kontaktzone ein den jeweiligen Stützstab (21) radial nach außen drängendes Keilgetriebepaar darstellt,
- wobei die Stützstäbe (21) an mindestens einem, am Gehäuse (10) gelagerten und in einer Sperrstellung (8) positionierten, Auslöseelement (82) - radial nach außen drückend - anliegen,
- wobei das Auslöseelement (82) - als Teil einer Auslöseeinheit (80) - durch Verschieben in eine - den Kolbenbetätigungsstempel (60) freigebende, keine abstützende Wirkung aufweisende - Auslösestellung (9) bringbar ist,
**dadurch gekennzeichnet, dass** der Stempelteller (73) zur Abstützung des Federenergiespeichers ist, dass der Durchmesser des Kolbenschiebers (76) etwas kleiner als der Innendurchmesser des Zylinders (101) der Zylinder-Kolben-Einheit (100) ist und dass der Durchmesser des Stempeltellers (73) einige Zehntel Millimeter kleiner als der Innendurchmesser des Gehäuses (10) ist, und
dass das Auslöseelement (82) eine außen am Gehäuse (10) gleitgelagerte Schiebehülse ist.

2. Einweginjektor gemäß Anspruch 1,
**dadurch gekennzeichnet,**
**dass** das der Kolbenbetätigungsstempel (60) an seiner - dem Federelement (50) abgewandten - Stirnfläche zumindest bereichsweise ebene Keilflächen oder bereichsweise einzelne kegelstumpfmantelförmige Flächen (74, 75) aufweist.

3. Einweginjektor gemäß Anspruch 1,
**dadurch gekennzeichnet,**
**dass** der Kolbenbetätigungsstempel (60) zusammen mit jedem einzelnen Stützstab (21) ein Schiebekeilgetriebe bildet, in dem eine axiale Federkraftrichtung in eine radiale Stützkraftrichtung umgelenkt wird.

4. Einweginjektor gemäß Anspruch 1,
**dadurch gekennzeichnet,**
**dass** der Kolbenbetätigungsstempel (60) im oberen Bereich des Kolbenschiebers (76) eine konische Verbreiterung (77) - zur knicksicheren Abstützung der Stützstäbe (21) in der Sperrstellung (8) - aufweist.

5. Einweginjektor gemäß Anspruch 1,
**dadurch gekennzeichnet,**
**dass** der einzelne Stützstab (21) an seinem freien Ende eine ebene, kegelstumpfmantelförmige oder sphärische Abstützfläche (23) aufweist.

6. Einweginjektor gemäß Anspruch 1,
**dadurch gekennzeichnet,**
**dass** das Auslöseelement (82) - zwischen seiner Sperrstellung (8) und seiner Auslösestellung (9) - einen geradlinigen, parallel zu einer Gehäusemittellinie (5) verlaufenden Verschiebeweg hat.

7. Einweginjektor gemäß Anspruch 1,
**dadurch gekennzeichnet,**
**dass** das Auslöseelement (82) in Kombination mit dem Gehäuse (10) und einer an ihm befestigten Abreißbanderole (90, 95) eine Auslöseeinheit (80) bildet.

## Claims

1. Disposable injector with a housing (10) in which or on which are arranged, in each case at least in some regions, at least one mechanical spring energy reservoir (50), at least one cylinder/piston unit (100) that can be filled at least temporarily with active substance, at least one piston-actuating ram (60) and at least one trigger unit (80),
- wherein the piston-actuating ram (60) is positioned between the spring energy reservoir (50) and the piston (111) of the cylinder/piston unit (100),
- wherein the spring energy reservoir (50) comprises at least one pre-tensioned spring element (50),
- wherein the spring-loaded piston-actuating ram (60) has a ram plate (73),
- wherein the spring-loaded piston-actuating ram (60) is supported on the housing (10) at the ram plate (73) via at least one support rod (21),
- wherein the individual support rod (21) is in each case integrally formed on the housing (10) and constitutes an elastic flexural beam (28),
- wherein the contact zone located between an individual support rod (21) and the ram plate (73) constitutes a wedge gear pairing that forces the respective support rod (21) radially outwards,
- wherein the support rods (21) bear on, and press radially outwards, at least one trigger element (82) mounted on the housing (10) and positioned in a blocking position (8),
- wherein the trigger element (82), as part of a trigger unit (80), can be brought by displacement to a triggering position (9) that releases the piston-actuating ram (60) and has no supporting action,
**characterized in that** the ram plate (73) is for supporting the spring energy reservoir,
**in that** the diameter of the piston slide (76) is slightly smaller than the internal diameter of the cylinder (101) of the cylinder/piston unit (100), and
**in that** the diameter of the ram plate (73) is a few tenths of a millimetre smaller than the internal diameter of the housing (10), and
**in that** the trigger element (82) is a slide sleeve mounted slidably on the outside of the housing (10).

2. Disposable injector according to Claim 1, **characterized in that** the piston-actuating ram (60), on its front face directed away from the spring element (50), has, at least in some regions, plane wedge surfaces or, in some regions, individual frustoconical surfaces (74, 75).

3. Disposable injector according to Claim 1, **characterized in that**, together with each individual support rod (21), the piston-actuating ram (60) forms a spline gear in which an axial spring force direction is converted into a radial supporting force direction.

4. Disposable injector according to Claim 1, **characterized in that** the piston-actuating ram (60) has, in the upper region of the piston slide (76), a conical widening (77) that safeguards the support rods (21) against buckling in the blocking position (8) .

5. Disposable injector according to Claim 1, **characterized in that** the individual support rod (21) has, at its free end, a plane, frustoconical or spherical support surface (23).

6. Disposable injector according, to Claim 1, **characterized in that** the trigger element (82), between its blocking position (8) and its triggering position (9), has a rectilinear path of travel extending parallel to a centre line (5) of the housing.

7. Disposable injector according to Claim 1, **characterized in that** the trigger element (82) forms a trigger unit (80) in combination with the housing (10) and with a tear-off banderole (90, 95) secured thereon.

## Revendications

1. Injecteur à usage unique comprenant un boîtier (10) dans lequel ou sur lequel est disposé à chaque fois au moins en partie au moins un accumulateur d'énergie mécanique à ressort (50), au moins une unité cylindre-piston (100) pouvant être remplie au moins temporairement de substance active, au moins un poinçon d'actionnement de piston (60) et au moins une unité de déclenchement (80),
- le poinçon d'actionnement de piston (60) étant positionné entre l'accumulateur d'énergie à ressort (50) et le piston (111) de l'unité cylindre-piston (100),
- l'accumulateur d'énergie à ressort (50) comprenant au moins un élément de ressort précontraint (50),
- le poinçon d'actionnement de piston (60) sollicité par ressort présentant un plateau de poinçon (73),
- le poinçon d'actionnement de piston (60) sollicité par ressort étant supporté sur le boîtier (10) au niveau du plateau de poinçon (73) par le biais d'au moins une barre de support (21),
- la barre de support individuelle (21) étant façonnée à chaque fois sur le boîtier (10) et constituant une poutre flexible élastique (28),
- la zone de contact située entre une barre de support individuelle (21) et le plateau de poinçon (73) constituant une paire de mécanismes à clavette poussant radialement vers l'extérieur la barre de support respective (21),
- les barres de support (21) s'appliquant - empressant radialement vers l'extérieur - contre au moins un élément de déclenchement (82) supporté sur le boîtier (10) et positionné dans une position de blocage (8),
- l'élément de déclenchement (82), en tant que partie d'une unité de déclenchement (80), pouvant être amené par déplacement dans une position de déclenchement (9) libérant le poinçon d'actionnement de piston (60), et ne présentant pas d'effet de support,
**caractérisé en ce que** le plateau de poinçon (73) est prévu pour supporter l'accumulateur d'énergie à ressort,
**en ce que** le diamètre du coulisseau de piston (76) est un peu plus petit que le diamètre intérieur du cylindre (101) de l'unité cylindre-piston (100) et **en ce que** le diamètre du plateau de poinçon (73) est plus petit d'un dixième de millimètre que le diamètre intérieur du boîtier (10), et
**en ce que** l'élément de déclenchement (82) est un manchon coulissant supporté de manière coulissante à l'extérieur sur le boîtier (10).

2. Injecteur à usage unique selon la revendication 1,
**caractérisé en ce que**
le poinçon d'actionnement de piston (60) présente au niveau de sa surface frontale opposée à l'élément de ressort (50) au moins en partie des surfaces de clavette planes ou au moins en partie des surfaces (74, 75) en forme d'enveloppe tronconique individuelles.

3. Injecteur à usage unique selon la revendication 1,
**caractérisé en ce que**
le poinçon d'actionnement de piston (60) conjointement avec chaque barre de support individuelle (21) forme un mécanisme à clavette coulissant dans lequel une direction de force de ressort axiale est déviée dans une direction de force de support radiale.

4. Injecteur à usage unique selon la revendication 1,
**caractérisé en ce que**
le poinçon d'actionnement de piston (60) présente dans la région supérieure du coulisseau de piston (76) un élargissement conique (77) pour le support empêchant la flexion des barres de support (21) dans la position de blocage (8).

5. Injecteur à usage unique selon la revendication 1,
**caractérisé en ce que**
la barre de support individuelle (21) présente au niveau de son extrémité libre une surface de support (23) plane, en forme d'enveloppe tronconique ou sphérique.

6. Injecteur à usage unique selon la revendication 1,
caractérisé-en ce que
l'élément de déclenchement (82) présente entre sa position de blocage (8) et sa position de déclenchement (9) une course de coulissement s'étendant en ligne droite, parallèlement à un axe médian du boîtier (5).

7. Injecteur à usage unique selon la revendication 1,
**caractérisé en ce que**
l'élément de déclenchement (82) forme, en combinaison avec le boîtier (10) et une banderole de déchirure fixée sur celui-ci (90, 95) une unité de déclenchement (80).
